Europäisches Patentamt

European Patent Office

Office européen des brevets

(11) Publication number: **0 322 020**
**A1**

## (12) EUROPEAN PATENT APPLICATION

(21) Application number: 88202825.1

(22) Date of filing: 09.12.88

(51) Int. Cl.4: **A61K 31/565**

(30) Priority: 22.12.87 US 136584

(43) Date of publication of application:
28.06.89 Bulletin 89/26

(84) Designated Contracting States:
AT BE CH DE ES FR GB GR IT LI NL SE

(71) Applicant: AKZO N.V.
Velperweg 76
NL-6824 BM Arnhem(NL)

(72) Inventor: Borah, Kripanath
34 Overlook Trail
Morris Plains, NJ 07950(US)
Inventor: Osinski, Patrick J.
17 Clark Street
Chatham, NJ 07928(US)
Inventor: Carmelitano, James J.
142-20 248th Street
Rosedale, NY 11422(US)

(74) Representative: Hermans, Franciscus G.M. et al
Patent Department AKZO N.V. Pharma
Division P.O. Box 20
NL-5340 BH Oss(NL)

(54) Pharmaceutical dosage unit for treating climacteric complaints and osteoporosis.

(57) Pharmaceutical dosage unit for oral application for treating or preventing climacteric complaints and symptoms, and/or osteoporosis in women comprising as active ingredients desogestrel and at least one alkali metal sulfate of a conjugated estrogen selected from the group consisting of estrone, equilin, 17α-dihydro-equilin, 17α-estradiol, equilenin, 17β-dihydro-equilin and 17β-dihydro-equilenin, and pharmaceutical preparations containing such dosage units.

EP 0 322 020 A1

# PHARMACEUTICAL DOSAGE UNIT FOR TREATING CLIMACTERIC COMPLAINTS AND OSTEOPOROSIS

## DESCRIPTION OF THE INVENTION

The present invention is concerned with an oral pharmaceutical dosage unit for the prevention or treatment of climacteric complaints and symptoms, and osteoporosis, which comprises at least one alkali metal sulfate of a conjugated estrogen selected from the group consisting of estrone, equilin, 17α-dihydro-equilin, 17α-estradiol, equilenin, 17β-dihydro-equilin, and 17β-dihydro-equilenin and a progestational compound. The present invention also relates to pharmaceutical preparations comprising one or more of these pharmaceutical dosage units.

## BACKGROUND OF THE INVENTION

A dosage unit for oral application for the prevention or treatment of climacteric complaints comprising at least one of the above estrogens has been disclosed in U.S. Patent 4,154,820. This U.S. patent further discloses the use of a progestin concomitantly or sequentially with the dosage unit comprising the estrogen.

European Patent Application 136,011 discloses many progestational compounds for use as agents for preventing or treating climacteric complaints together with an estrogen.

Many pharmaceutical preparations used in practice exhibit side-effects, such as a negative influence on the high density lipid (HDL)- and triglyceride concentration in the blood and irregular withdrawal bleeding.

Surprisingly, we found that a combination of at least one of the above estrogens and desogestrel does not exhibit these drawbacks. The withdrawal bleedings is absent or regular, which avoids the excessive stimulation of the endometrium. Furthermore, metabolic parameters like HDL- and triglyceride concentration remain practically at the same level as if the dosage unit according to the present invention was not administered. Accordingly, the composition of the present invention is very suitable for the prevention or treatment of climacteric complaints, especially for the prevention or treatment of vasomotoric complaints and osteoporosis.

Use of the above estrogens and desogestrel sequentially or concomitantly appear to synergistically increase the beneficial effects while decreasing the risks. By applying an effective amount of the above estrogens bone loss is prevented and climacteric complaints are suppressed. By applying an effective amount of desogestrel during a certain period, side effects that would otherwise be caused by the administration of the above estrogens are avoided without adding other side effects, such as androgen side effects.

Desogestrel has been disclosed in U.S. Patent 3,927,046. However, the use of this compound for the prevention or treatment of climacteric complaints and/or osteoporosis in women has not been disclosed.

## SUMMARY OF THE INVENTION

The present invention relates to a pharmaceutical composition in the form of a dosage unit to be administered orally for the prevention or treatment of climacteric complaints and/or osteoporosis in women, comprising at least one alkali metal sulfate of a conjugated estrogen selected from the group consisting of estrone, equilin, 17α-dihydro-equilin, 17α-estradiol, equilenin, 17β-dihydro-equilin and 17β-dihydro-equilenin and desogestrel as the active ingredients. It also relates to a pharmaceutical preparation comprising one or more of these pharmaceutical dosage units.

In the preferred embodiment the estrogen employed is a combination of sodium estrone sulfate, sodium equilin sulfate and 17α-dihydro-equilin sodium sulfate, more preferably in a weight ratio of about 6:3:1.

A dosage unit preferably comprises 0.1-3.0 mg and more preferably 0.2-2.50 mg estrogen, and preferably 25-300 µg and more preferably 50-250µg desogestrel.

The simplest way to prevent or to treat climacteric complaints and/or osteoporosis in women is to administer one dosage unit according to the present invention every day without interruption. For this purpose dosage units, each containing the same amount of estrogen and the same amount of desogestrel, may be packaged in a simple way without indicating any particular order in which the dosage units should be taken. A suitable way of packaging is in a tube or a bottle. In this case the preparation does not contain other dosage units except those according to the present invention.

If regular withdrawal bleeding is preferred, e.g. after one, two or three months, a pharmaceutical preparation comprising at least two phases may be used, the first phase consists of at least 11, preferably 11-100 and more preferably 11-15 dosage units comprising the estrogen compound but no desogestrel, and the second phase comprising 11-15 dosage units according to the present invention including desogestrel. Preferably, the preparation comprises two or three phases.

If the preparation comprises two phases, each phase more preferably comprises 13-15, most preferably 14 successive dosage units.

A preparation comprising three-phases preferably has a first and a second phase of 11-13 dosage units and a third phase consisting of a period wherein no dosage units are taken or a third phase comprising placebo dosage units. The third phase comprises 2-6 days without dosage units or 2-6 placebos. Preferably, the third phase comprises 4 days without dosage units or 4 placebos. The use of placebos is preferred in view of its convenience to the user.

For preparations comprising two or more phases, dosage units are taken one each day in the order indicated on the packaging or in the enclosed leaflet.

In preparations comprising two or more phases the amount of estrogen and the amount of desogestrel in each dosage unit in which desogestrel is present are preferably both the same for ease of manufacture. However, the amounts of each ingredient can vary within the limits set forth above.

Active ingredients in addition to those already mentioned in the dosage units and preparations according to the present invention may be included. In the preferred embodiment, however, dosage units and preparations exclusively comprising the estrogen component and desogestrel as the active ingredients are preferred.

For purposes of describing the invention, except in the examples, the term "dosage units" is meant to indicate tablets or other conventional pills, capsules, coated tablets and granules. The oral dosage units, which may have a total weight of 50-300 mg, are obtained by mixing the desired quantity of desogestrel and estrogen using the normal pharmaceutically acceptable aids such as fillers, binders, disintegrating agents, coloring agents, flavors, anti-oxidants and lubricants, and bringing the mixture into the form of a pharmaceutical moulding, which may be coated or used for filling capsules.

Dosage units according to the present invention may be prepared in the same way as the dosage units in U.S. Patent 4,154,820, which is included herein by reference. Of course, desogestrel should be added as well. Dosage units comprising estrogens only and placebos may be prepared in the same way.

It is recommended that the placebos and the tablets in the other phases be distinguished from each other by giving them different shapes and/or colors.

Date indications should be provided on the packages in which preparations according to the invention are packed, indicating the date on which each dosage unit should be taken.

The preparation can be packed in a tube or box or in strip packaging. In the event a small box is used, which can have circular, square or other shape, the tablets are accommodated separately therein, usually along the periphery of the box, with a series of date indications, either adjustable or not, corresponding to the days on which each of the tablets is to be taken marked on the box.

Another practical form of packaging is strip packaging or push-through packaging whereby each tablet is sealed in a separate compartment and where, on the strip or the packaging, date indications or other sorts of indications are provided to show the sequence in which the tablets should be taken.

The invention will now be explained with the aid of the following examples, which are directed to specific preferred embodiments of the invention and are to be construed as illustrative but not limiting as to the scope of the invention defined in the appended claims.

## EXAMPLE 1

Tablets, containing the amounts of conjugated estrogens (sodium estron sulfate, sodium equilin sulfate and sodium 17α-dihydro-equilin sulfate, weight ratio 6:3:1), desogestrel, tromethamine, dl-α-tocopherol and sodium citrate given in Table 1 and 25.0 mg starch, 60.0 mg microcrystalline cellulose, 4.0 mg hydroxypropyl methylcellulose, 6.0 mg stearic acid, 1.5 mg silicon dioxide, 2.0 mg magnesium stearate and an amount of lactose sufficient to make the total weight of the completed tablet 200.0 mg, were prepared by blending together the lactose, starch, microcrystalline cellulose and hydroxypropyl methylcellulose;

granulating this blend by adding alcoholic solution of dl-α-tocopherol and 5 desogestrel followed by adding an aqueous solution of conjugated estrogens, tromethamine and sodium citrate; drying; milling; lubricating with stearic acid, magnesium stearate and silicon dioxide and compressing into tablets.

Table 1

| Tablet | 1 | 2 | 3 | 4 | 5 | 6 | 7 | 8 | 9 |
|---|---|---|---|---|---|---|---|---|---|
| conjugated estrogens (mg) | 0.30 | 0.30 | 0.30 | 0.625 | 0.9 | 1.25 | 2.5 | 0.625 | 0 |
| desogestrel (mg) | 0.075 | 0.100 | 0.150 | 0.150 | 0.150 | 0.150 | 0.150 | 0 | 0 |
| tromethamine (mg) | 0.20 | 0.20 | 0.20 | 0.417 | 0.6 | 0.833 | 1.667 | 0.417 | 0 |
| dl-ã-tocopherol (mg) | 0.1 | 0.1 | 0.1 | - | 0.1 | 0.1 | 0.2 | - | - |
| sodium citrate (mg) | - | - | - | 0.6 | - | - | - | 0.6 | 0.6 |

Example 2

Pharmaceutical preparations from dosage units prepared in Example 1:

Table 2

| Pharmaceutical preparation | 1 | 2 | 3 | 4 | 5 | 6 |
|---|---|---|---|---|---|---|
| 1st phase | 12 x tablet 8 | 13 x tablet 8 | 14 x tablet 8 | 70 x tablet 8 | 12 x tablet 8 | 60 x tablet 6 |
| 2nd phase | 12 x tablet 4 | 12 x tablet 4 | 14 x tablet 4 | 14 x tablet 4 | 12 x tablet 4 | - |
| 3rd phase | 4 x tablet 9 | 5 x tablet 9 | - | - | 4 x nothing | - |
| "-" means not applicable | | | | | | |
| "nothing" means that the 3rd phase consists of a period during which no tablets are taken. | | | | | | |

## Claims

1. A pharmaceutical dosage unit for oral administration for treating or preventing climacteric complaints and/or osteoporosis in women, comprising as active ingredients desogestrel and at least one alkali metal sulfate of a conjugated estrogen selected from the group consisting of estrone, equilin, 17α-dihydro-equilin, 17α-estradiol, equilenin, 17β-dihydro-equilin and 17β-dihydro-equilenin.

2. The pharmaceutical dosage unit according to claim 1, comprising 0.1-0.3 mg of estrogen and 25-300 μg of desogestrel.

3. A pharmaceutical preparation comprising a plurality of dosage units according to claim 1.

4. A pharmaceutical preparation consisting of a plurality of dosage units according to claim 1.

5. The pharmaceutical preparation according to claim 3, comprising at least 22 dosage units in at least two phases, the first phase comprising at least 11 and the second phase comprising 11-15 successive dosage units, the dosage units of the first phase each comprising as active ingredients at least one alkali metal sulfate of a conjugated estrogen selected from the group consisting of estrone, equilin, 17α-dihydro-equilin, 17α-estradiol, equilenin, 17β-dihydroequilin and 17β-dihydro-equilenin and the dosage units of the second phase each being a dosage unit according to claim 1.

6. The pharmaceutical preparation according to claim 5, wherein the total number of dosage units is 22-30 and the number of dosage units in the first phase is 11-15.

4

7. The pharmaceutical preparation according to claim 5, wherein the preparation comprises two phases, each phase comprising 14 dosage units.

The pharmaceutical preparation according to claim 5, wherein the preparation comprises three phases, the first and second phase comprising 11-13 dosage units and the third phase comprising 2-6 placebo dosage units.

9. The pharmaceutical preparation according to claim 3, wherein the amount of estrogen in each dosage unit containing estrogen is the same.

10. The pharmaceutical preparation according to claim 3, wherein the amount of desogestrel in each dosage unit containing desogestrel is the same.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 154 820 (J.R.A. SIMOONS)<br>* Column 5, lines 30-61 *<br>--- | 1-10 | A 61 K 31/565 |
| Y | UNLISTED DRUGS, vol. 36, no. 6, June 1984, page 108,p, Chatham, New Jersey, US<br>* Page 108,p: "Neophasic" *<br>--- | 1-10 | |
| D,A | EP-A-0 136 011 (E.R. PLUNKETT)<br>----- | 1-10 | |

| | |
|---|---|
| | TECHNICAL FIELDS SEARCHED (Int. Cl.4) |
| | A 61 K |

The present search report has been drawn up for all claims

| Place of search | Date of completion of the search | Examiner |
|---|---|---|
| THE HAGUE | 05-04-1989 | BRINKMANN C. |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
--------------------------------------------------
& : member of the same patent family, corresponding
   document

EPO FORM 1503 03.82 (P0401)